# EUROPEAN PATENT SPECIFICATION

(11) **EP 3 305 340 B1**
(45) Date of publication and mention of the grant of the patent: **24.04.2024**
(21) Application number: 16877806.6
(22) Date of filing: 24.12.2016
(51) Int. Cl.: A61L 27/36, A61L 27/56, A61L 27/50

(54) **CELL-GROWING SCAFFOLD HAVING STRUCTURE MEMORY PROPERTY**
ZELLWACHSTUMSGERÜST MIT STRUKTURGEDÄCHTNIS
ÉCHAFAUDAGE DE CROISSANCE CELLULAIRE À PROPRIÉTÉ DE MÉMOIRE DE STRUCTURE

(30) Priority: 25.12.2015 CN 201510986268
(43) Date of publication of application: 11.04.2018
(73) Proprietor: Beijing Ruijian Gaoke Biotechnology Co., Ltd., Beijing 102200 (CN)
(72) Inventor: SUN, Wenquan, Beijing 102200 (CN)
(74) Representative: Modiano, Micaela Nadia
(86) International application number: PCT/CN2016/111925
(87) International publication number: WO 2017/107996

(56) References cited:
- WO-A1-2012/006390
- CN-A- 1 407 002
- CN-A- 103 432 627
- CN-A- 104 107 456
- CN-A- 104 144 715
- CN-A- 105 169 483
- US-A1- 2007 248 575
- US-A1- 2012 263 763

## Description

### Technical Field

The present invention relates to a cell growth scaffold applied in the fields of material science, tissue engineering and regenerative medicine, in particular, to a cell growth scaffold having a structural memory property.

### Background of the Related Art

Since lesion, trauma and surgery often cause defects of soft tissues of a human body, regrowth of the soft tissues required for human bodies *per se* is a problem that needs to be solved by modern medicine. Various filling materials and dressings for repair of soft tissue defects, including a hydrogel and a porous sponge-type filling material prepared based on artificially synthesized polymer materials and natural biological materials have been developed at home and abroad. The collagen purified by acid treatment or enzyme treatment with animal tissues as raw materials, can be prepared into a collagen hydrogel, and the stability of the collagen in a hydrogel formulation can be enhanced by an appropriate chemical crosslinking treatment, so that the collagen is not easy to be degraded *in vivo.* The collagen suspension or hydrogel can be prepared into a porous collagen sponge material by freeze-drying, and by further chemical crosslinking fixation, not only the stability of the collagen in the sponge material can be increased, but also the porous structural property of the collagen sponge material can be retained. The porous collagen sponge material is widely used in hemostasis of wounds, filling of tissue defect sites and drug carrier, etc. in the clinical medicine.

Chinese patent literatures CN1235947C, CN101549171B provide specific methods for preparing such collagen sponge materials, respectively. Wherein, the technical solution provided in the patent literature CN1235947C is soaking animal tissues (skin, cartilage, sinew, tendon, etc.) containing rich collagen with a hydrochloric acid or acetic acid solution at room temperature, obtaining an extracellular collagen frame of the animal tissues after enzymatic digestion, obtaining a collagen suspension by grinding and homogenizing treatment, and placing the collagen suspension into a mold, to prepare a collagen sponge filler by extrusion forming and freeze-drying, in which treatment with a chemical crosslinker is used before or after the freeze-drying. Patent literature CN101549171B proposes that, after the high purity II-type collagen solution is extracted, the II-type collagen solution is concentrated with polyethylene glycol, and then the concentrated II-type collagen is crosslinked with a crosslinker containing carbodiimide and N-hydroxysuccinimide, and freeze-dried to produce a collagen sponge.

The above technical solutions both obtain a collagen sponge by means of chemical crosslinking, but the shortcomes are obvious: the collagen sponge prepared by acidification and enzymatic digestion has drawbacks such as poor stability, lack of biological activity and rapid degradation.

In addition, in the technical field, other biological macromolecule materials such as sodium hyaluronate, chitosan, chitin, chondroitin sulfate are also added in the preparation of a collagen sponge to prepare a composite sponge material and the biological properties of the collagen sponge are improved. For example, Chinese patent literature CN101862475B discloses a technical solution that sodium hyaluronate is added into the collagen solution, followed by chemical crosslinking to prepare a composite collagen material. In Chinese patent literature CN103007336A, chitosan is added into the fishskin collagen, freeze-dried, crosslinked, and then freeze-drying is again performed to prepare a fishskin collagen-based composite sponge. Although the stability of the collagen sponge was enhanced by chemical crosslinking treatment, and the collagen sponge composite material containing other biological macromolecules such as hyaluronic acid and chitosan has better biological activity, the stability of these sponge materials is still significantly decreased after radiation sterilization, and a relatively severe inflammation is prone to occur in large-area soft tissue filling or wound repair.

While the above technical solution enhances the stability of the collagen sponge by way of a chemical crosslinking, and the collagen sponge composite material containing other biological macromolecules such as hyaluronic acid and chitosan has better biological activity, there is still a problem in practice that the stability of these sponge materials is significantly decreased after the radiation sterilization, especially, a relatively severe inflammation is prone to occur in large-area soft tissue filling or wound repair.

US patent literature US2012/0263763A1 describes a fibrotic acellular tissue matrix-based sponge material. A method for preparing this material is to prepare a tissue matrix sponge material with pigskin as a raw material, followed by fat removal, ultracryotomy, decellularization and antigen removal, cleaning, fibrotic homogenization, virus inactivation, sterilization, and freeze-drying the acellular matrix suspension, the freeze-dried sponge material can be sterilized with oxirane or radiation. The positive effect generated from this method is to make this extracellular tissue matrix material better retain the basic structural features of the original tissue matrix without using chemical crosslinking; in addition to the collagen component, other important extracellular tissue matrix components, such as elastin, fibrous protein and proteoglycan, are also contained; with no acid treatment and enzymatic digestion treatment in the preparation process, this sponge material has good biocompatibility, supports the rapid growth of host cells, and has better collagen stability, thereby reducing inflammatory response. However, the existing problem is that this sponge material obtained by this method has poor structure strength and elasticity, is prone to be crushed and deformed after being pressed, and cannot maintain the original material structure and recover to the original shape.

By summarizing the technical methods disclosed in the above literatures, the significant problem is: no matter whether to use the collagen hydrogel purified after acidolysis or enzymolysis, or to use the tissue matrix material suspension homogenized after decellularization, when a porous collagen sponge material is prepared, the prior art is firstly freeze-drying the hydrogel or suspension, followed by further chemically crosslinking treatment to enhance the mechanical strength of the material and increase the stability of the collagen in the sponge material, the chemical crosslinking treatment makes the tissue matrix material lose many excellent natural characteristics inherently possessed by the porous cell growth scaffold.

The patent document US2007/248575A discloses methods of making a bone graft composition.

### Content of the Invention

The technical problem to be solved in the present invention is to provide a cell growth scaffold having a structural memory property that not only has an excellent biocompatibility and complete biodegradability but also supports the growth of cells and the growth of tissues and organs *in vivo* and *in vitro.*

Therefore, the technical solution solving said problem in the present invention is: a cell growth scaffold having a memory property structure, comprising a matrix material for cell growth, characterised in that, said scaffold is formed by mixing, in respective dry weight mass proportions, a micro-fibrous or flocculent acellular tissue matrix material having a fibre diameter of 2-250 microns and a length of 100-3000 microns with an acidification-treated, hydrogel-like acellular tissue matrix material having a particle diameter of 2-150 microns, followed by injection molding, freezing and extruding, re-interweaving and radiation processes; wherein, the matrix material for cell growth is a matrix material of said cell growth scaffold, the content of the total acellular tissue matrix materials in the matrix material of said cell growth scaffold is 10~100 mg/cm³, the total voidage is 90~99%, and, the voidage of the matrix material with particles larger than 25 microns is 80~98%.

Preferably, the micro-fibrous or flocculent acellular tissue matrix material in said scaffold is 60%~90% by the dry weight mass proportion, and said acidification-treated, hydrogel-like acellular tissue matrix material is 40%~10% by the dry weight mass proportion.

Preferably, said injection molding process is that the micro-fibrous or flocculent acellular tissue matrix material and said acidification-treated, hydrogel-like acellular tissue matrix material are uniformly mixed into a suspension and then injecting it into a mold.

Preferably, said freezing process is that the mixed suspension in the mold is frozen under the condition of -20 °C into an ice crystal, which is extruded, re-interweaved and radiation-treated by gamma ray.

Preferably, said extruding and re-interweaving processes are that the ice crystal is extruded to re-interweave the micro-fibrous or flocculent acellular tissue matrix material and the acidification-treated, hydrogel-like acellular tissue matrix granular material in the suspension together, to produce a porous cell growth scaffold material having a stable three-dimensional structure and a structure memory property.

Preferably, said scaffold molding process is that the porous cell growth scaffold material having a stable three-dimensional structure and a structure memory property is produced into scaffold products in various shapes.

Preferably, said freezing process can be a process for freeze thawing the mixed suspension in the mold for at least two times.

As compared with the prior art, the positive effects possessed in the present invention are obvious: this cell growth scaffold is a porous cell growth scaffold having no chemical cross-linking and having biological activity, a stable three-dimensional structure and a structure memory feature, which is a porous material having special properties prepared by mixing, in a certain proportion, a fibrous or flocculent tissue material with an acidified hydrogel material, followed by freezing/thawing and extruding and radiation processes. This scaffold not only has an excellent biocompatibility and complete biodegradability but also supports the growth of cells as well as the growth of tissues and organs *in vivo* and *in vitro,* thereby being suitable for the repair of human soft tissue traumas and defects.

### Description of the Drawings

FIG. 1 is a schematic structural diagram of one example of the present invention;
FIG. 2 are thermograms of differential scanning calorimetry (DSC) of the scaffold product in FIG. 1;
FIG. 3 is a curve demonstrating the relationship between the stability and the particle diameter of two materials forming the scaffold product;
FIG. 4 are thermograms of differential scanning calorimetry of the scaffold products in FIG. 1.

### Detailed Description of Embodiments

The present invention relates to a cell growth scaffold having a structural memory property, with structural features of said cell growth scaffold described in the following examples.

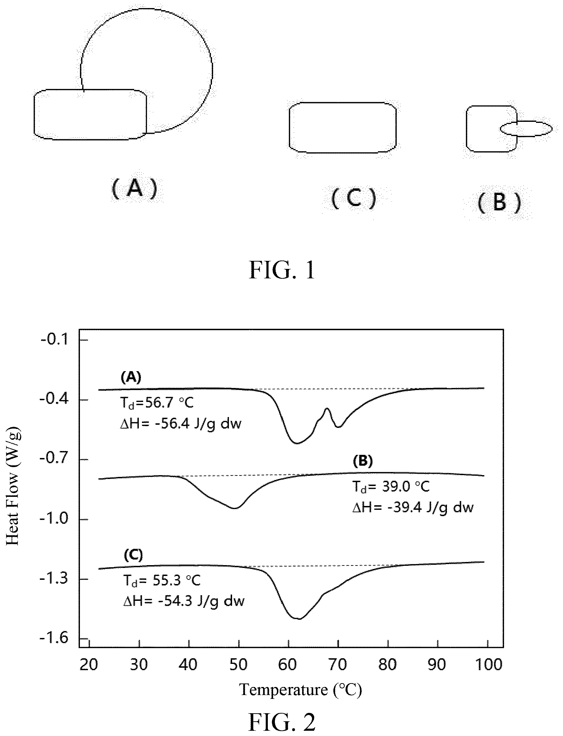

For example, FIG. 1 shows the morphological structure of some products involved in the present invention and the description thereof, wherein, (A) in the figure is a disc-shaped and cylinder-shaped cell growth scaffold; (B) in the figure is a pillar-shaped cell growth scaffold with a diameter of 1.8 cm and a height of 3.0 cm, which is in the contraction state under a pressure action (pinched by fingers); (C) in the figure is the scaffold which is recovered to the original, stable three-dimensional structure and shape after the pressure (pinched by fingers) is released.

For example, FIG. 2 are thermograms of differential scanning calorimeter (DSC) of a tissue matrix material after being decellularized by a 0.5% sodium deoxycholate solution; wherein, (A) in the figure is a micro-fibrous or flocculent acellular tissue matrix material; (B) in the figure is an acellular tissue matrix hydrogel-like particles after being treated by a 50 mM acetic acid solution; (C) in the figure is a cell growth scaffold prepared from 70% micro-fibrous acellular tissue matrix material and 30% acidification-treated hydrogel-like particles.

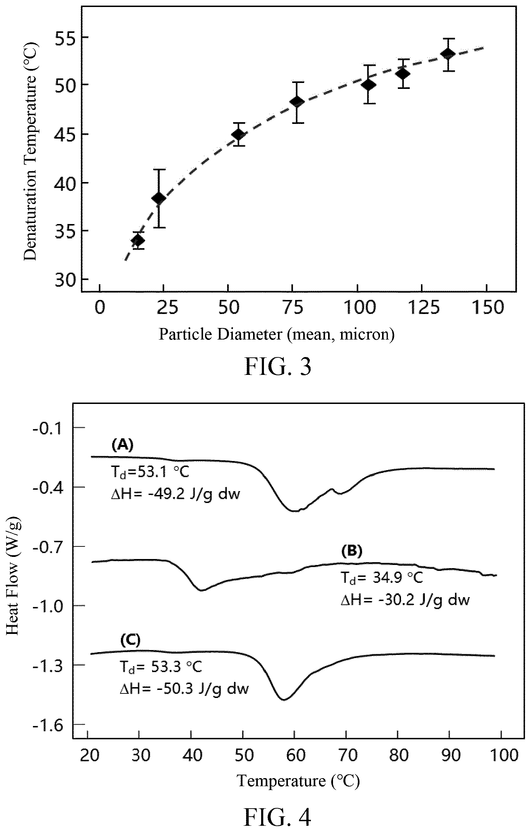

For example, FIG. 3 is a curve showing the relationship between the stability and the particle diameter of the acellular tissue matrix hydrogel-like particles after the decellularization treatment by a 0.5% sodium deoxycholate solution and the treatment by 50 mM acetic acid.

For example, FIG. 4 are thermograms of differential scanning calorimeter (DSC) of the tissue matrix material after being decellularized by a 0.5% lauryl sodium sulfate solution; wherein, (A) in the figure is a micro-fibrous or flocculent acellular tissue matrix material; (B) in the figure is an acellular tissue matrix hydrogel-like particles after being treated by a 50 mM acetic acid solution; (C) in the figure is a cell growth scaffold prepared from 80% micro-fibrous acellular tissue matrix material and 20% acidification-treated hydrogel-like particles.

The above figures illustrate a cell growth scaffold having a structural memory property that can be widely used in the fields such as general surgery, orthopedics, plastic surgery, tissue engineering and regenerative medicine.

The so-called matrix material for cell growth is a material consisting of two acellular tissue matrix materials with different properties in a certain proportion, in particular, the acellular tissue matrix material is obtained from, but not limited to, skin, cartilage, blood vessel, meniscus, stomach, small intestine, large intestine, diaphragm, tendon, ligament, nervous tissue, bladder and urethra, etc. of the human body or animal. The tissue being rich in collagen is cut and separated out, and is obtained in little pieces or little sheets that are cut into 1-20 mm.

After two acellular tissue matrix materials having different properties are obtained, the cell growth scaffold having structural memory property can be produced. For example, firstly, the tissue structure problem of the biological cell growth scaffold is solved, said scaffold in this example is formed by mixing, in respective dry weight mass proportions, a micro-fibrous or flocculent acellular tissue matrix material having a fibre diameter of 2-250 microns and a length of 100-3000 microns with an acidification-treated, hydrogel-like acellular tissue matrix material having a particle diameter of 2-150 microns, followed by injection molding, freezing, extruding, radiation, re-interweaving processes; wherein, the matrix material for cell growth is a matrix material of said cell growth scaffold, the content of the total acellular tissue matrix materials in the matrix material of said cell growth scaffold should be defined within a range of 10~100 mg/cm³, with the total voidage of 90~99%, and, the voidage of the matrix material with particles larger than 25 microns is in the range of 80-98%. During the production of the cell growth scaffold, the preferred solution is adjusting the ratio of the micro-fibrous or flocculent acellular tissue matrix material to be 60%~90% by the dry weight mass proportion, and the ratio of the acidification-treated, hydrogel-like acellular tissue matrix material to be 40%~10% by the dry weight mass proportion, in the scaffold; in the injection molding process, the micro-fibrous or flocculent acellular tissue matrix material and the acidification-treated, hydrogel-like acellular tissue matrix material should be uniformly mixed into a suspension and then injected into a mold; in the freezing process, the mixed suspension in the mold can be produced into an ice crystal only under the condition of -20 °C and after the radiation treatment by gamma ray, or a freezing procedure is finished by a process for freeze-thawing the mixed suspension in the mold for at least two times; in extruding and re-interweaving processes, the ice crystal should be extruded to re-interweave the micro-fibrous or flocculent acellular tissue matrix material and the acidification treated, hydrogel-like acellular tissue matrix granular material in the suspension together to produce a porous cell growth scaffold material having a stable three-dimensional structure and a structure memory property. Only after undergoing the above processes can a porous cell growth scaffold material having a stable three-dimensional structure and a structure memory feature be produced into scaffold products in various shapes.

## Claims

1. A cell growth scaffold having a memory property structure, comprising a matrix material for cell growth, **characterized in that**, said scaffold is formed by mixing, in respective dry weight mass proportions, a micro-fibrous or flocculent acellular tissue matrix material having a fibre diameter of 2-250 microns and a length of 100-3000 microns with an acidification-treated, hydrogel-like acellular tissue matrix material having a particle diameter of 2-150 microns, followed by injection molding, freezing and extruding, re-interweaving and radiation processes; wherein, the matrix material for cell growth is a matrix material of said cell growth scaffold, the content of the total acellular tissue matrix materials in the matrix material of said cell growth scaffold is 10~100 mg/cm³, the total voidage is 90~99%, and, the voidage of the matrix material with particles larger than 25 microns is 80-98%.

2. The cell growth scaffold having the memory property structure according to claim 1, wherein, the micro-fibrous or flocculent acellular tissue matrix material in said scaffold is 60%~90% by the dry weight mass proportion, and said acidification-treated, hydrogel-like acellular tissue matrix material is 40%~10% by the dry weight mass proportion.

3. The cell growth scaffold having the memory property structure according to claim 1, wherein, said injection molding process is that the micro-fibrous or flocculent acellular tissue matrix material and said acidification-treated, hydrogel-like acellular tissue matrix material are uniformly mixed into a suspension and then injecting it into a mold.

4. The cell growth scaffold having the memory property structure according to claim 1, wherein, said freezing process is that the mixed suspension in the mold is frozen under the condition of -20 °C into an ice crystal, which is extruded, re-interweaved and radiation-treated by gamma ray.

5. The cell growth scaffold having the memory property structure according to claim 1, wherein, said extruding and re-interweaving processes are that the ice crystal is extruded to re-interweave the micro-fibrous or flocculent acellular tissue matrix material and the acidification-treated, hydrogel-like acellular tissue matrix granular material in the suspension together, to produce a porous cell growth scaffold material having a stable three-dimensional structure and a structure memory property.

6. The cell growth scaffold having the memory property structure according to claim 1, wherein, said scaffold molding process is that the porous cell growth scaffold material having a stable three-dimensional structure and a structure memory property is produced into scaffold products in various shapes.

7. The cell growth scaffold having the memory property structure according to claim 4, wherein, said freezing process can be a process for freeze thawing the mixed suspension in the mold for at least two times.

## Patentansprüche

1. Ein Zellwachstumsgerüst mit Strukturgedächtnis, das ein Matrixmaterial für Zellwachstum umfasst; **dadurch gekennzeichnet, dass** das Gerüst gebildet wird durch Mischen, in entsprechenden Trockenmasseverhältnissen, eines mikrofaserigen oder flockenförmigen azellulären Gewebematrixmaterials, das einen Faserdurchmesser von 2-250 Mikron und eine Länge von 100-3000 Mikron hat, mit einem angesäuerten, hydrogelartigen azellulären Gewebematrixmaterial, das einen Partikeldurchmesser von 2-150 Mikron hat, gefolgt von Spritzgieß-, Gefrier- und Extrudier-, Neuverflechtungs- und Bestrahlungsprozessen; wobei das Matrixmaterial für Zellwachstum ein Matrixmaterial des Zellwachstumsgerüsts ist; wobei der Gesamtgehalt an azellulärem Gewebematrixmaterial im Matrixmaterial des Zellwachstumsgerüsts 10-100 mg/cm³ beträgt, die Gesamtporosität 90-99% beträgt und die Porosität des Matrixmaterials mit Partikeln, die größer sind als 25 Mikron, 80-98% beträgt.

2. Das Zellwachstumsgerüst mit Strukturgedächtnis gemäß Anspruch 1, wobei das mikrofaserige oder flockenförmige azelluläre Gewebematrixmaterial in dem Gerüst 60%-90% des Trockenmasseverhältnisses beträgt und das angesäuerte, hydrogelartige azelluläre Gewebematrixmaterial 40%-10% des Trockenmasseverhältnisses beträgt.

3. Das Zellwachstumsgerüst mit Strukturgedächtnis gemäß Anspruch 1, wobei der Spritzgießprozess darin besteht, dass das mikrofaserige oder flockenförmige azelluläre Gewebematrixmaterial und das angesäuerte, hydrogelartige azelluläre Gewebematrixmaterial einheitlich in eine Suspension hineingemischt und dann in eine Form eingespritzt werden.

4. Das Zellwachstumsgerüst mit Strukturgedächtnis gemäß Anspruch 1, wobei der Gefrierprozess darin besteht, dass die gemischte Suspension in der Form unter Bedingungen von -20°C zu einem Eiskristall gefroren wird, welcher extrudiert, neu verflochten und mit Gammastrahlen bestrahlt wird.

5. Das Zellwachstumsgerüst mit Strukturgedächtnis gemäß Anspruch 1, wobei die Extrudier- und Neuverflechtungs-Prozesse darin bestehen, dass das Eiskristall extrudiert wird, um das mikrofaserige oder flockenförmige azelluläre Gewebematrixmaterial und das körnige, angesäuerte, hydrogelartige, azelluläre Gewebematrixmaterial in der Suspension miteinander neu zu verflechten, um ein poröses Zellwachstumsgerüstmaterial zu erzeugen, das eine stabile dreidimensionale Struktur und ein Strukturgedächtnis hat.

6. Das Zellwachstumsgerüst mit Strukturgedächtnis gemäß Anspruch 1, wobei der Gerüstformprozess darin besteht, dass aus dem porösen Zellwachstumsgerüstmaterial mit stabiler dreidimensionaler Struktur und Strukturgedächtnis Gerüstprodukte mit verschiedenen Formen produziert werden.

7. Das Zellwachstumsgerüst mit Strukturgedächtnis gemäß Anspruch 4, wobei der Gefrierprozess ein Prozess mindestens zweifachen Ausfrierens der gemischten Suspension in der Form sein kann.

## Revendications

1. Échafaudage de croissance cellulaire ayant une structure à propriété de mémoire, comprenant un matériau de matrice pour croissance cellulaire, **caractérisé en ce que** ledit échafaudage est formé par mélange, en proportions en masse en poids sec respectives, d'un matériau acellulaire de matrice tissulaire floculant ou micro-fibreux ayant un diamètre de fibre de 2 - 250 micromètres et une longueur de 100 - 3 000 micromètres avec un matériau acellulaire de matrice tissulaire de type hydrogel, traité par acidification, ayant un diamètre de particule de 2 - 150 micromètres, suivi des processus de moulage par injection, congélation et extrusion, re-entrelacement et irradiation ; le matériau de matrice pour croissance cellulaire étant un matériau de matrice dudit échafaudage de croissance cellulaire, la teneur en la totalité des matériaux acellulaires de matrice tissulaire du matériau de matrice dudit échafaudage de croissance cellulaire valant 10 - 100 mg/cm³, la proportion totale de vides étant de 90-99 % et la proportion de vides du matériau de matrice comportant des particules de taille supérieure à 25 micromètres étant de 80 - 98 %.

2. Échafaudage de croissance cellulaire ayant la structure à propriété de mémoire selon la revendication 1, dans lequel le matériau acellulaire de matrice tissulaire floculant ou micro-fibreux dans ledit échafaudage constitue 60 % - 90 % en proportion de masse en poids sec, et ledit matériau acellulaire de matrice tissulaire de type hydrogel, traité par acidification, constitue 40 % - 10 % en proportion de masse en poids sec.

3. Échafaudage de croissance cellulaire ayant la structure à propriété de mémoire selon la revendication 1, dans lequel ledit processus de moulage par injection consiste en ce que le matériau acellulaire de matrice tissulaire floculant ou micro-fibreux et ledit matériau acellulaire de matrice tissulaire de type hydrogel, traité par acidification sont mélangés de façon homogène en une suspension et ensuite injectés dans un moule.

4. Échafaudage de croissance cellulaire ayant la structure à propriété de mémoire selon la revendication 1, dans lequel ledit processus de congélation consiste en ce que la suspension mélangée est congelée dans le moule dans la condition de -20 °C en un cristal de glace qui est extrudé, re-entrelacé et traité par irradiation par rayonnement gamma.

5. Échafaudage de croissance cellulaire ayant la structure à propriété de mémoire selon la revendication 1, dans lequel lesdits processus d'extrusion et de re-entrelacement consistent en ce que le cristal de glace est extrudé pour re-entrelacer entre eux dans la suspension le matériau acellulaire de matrice tissulaire floculant ou micro-fibreux et le matériau granulaire acellulaire de matrice tissulaire de type hydrogel, traité par acidification, pour produire un matériau poreux d'échafaudage de croissance cellulaire ayant une structure tridimensionnelle stable et une propriété de mémoire structurale.

6. Échafaudage de croissance cellulaire ayant la structure à propriété de mémoire selon la revendication 1, dans lequel ledit processus de moulage d'échafaudage consiste en ce que le matériau poreux d'échafaudage de croissance cellulaire ayant une structure tridimensionnelle stable et une propriété de mémoire structurale est produit en produits d'échaudage en diverses formes.

7. Échafaudage de croissance cellulaire ayant la structure à propriété de mémoire selon la revendication 4, dans lequel ledit processus de congélation peut être un processus pour congeler-décongeler au moins deux fois dans le moule la suspension mélangée.
